(19) Europäisches Patentamt
European Patent Office
Office européen des brevets

(11) Veröffentlichungsnummer: **0 093 875**
**B1**

(12) # EUROPÄISCHE PATENTSCHRIFT

(45) Veröffentlichungstag der Patentschrift: **04.11.87**

(51) Int. Cl.⁴: **C 01 B 13/02,** C 01 B 11/10, A 61 K 33/40

(21) Anmeldenummer: **83103345.1**

(22) Anmeldetag: **06.04.83**

(54) **Stabilisierter aktivierter Sauerstoff und Arzneimittel, die diesen stabilisierten aktivierten Sauerstoff enthalten.**

(30) Priorität: **10.04.82 DE 3213389**

(43) Veröffentlichungstag der Anmeldung: **16.11.83 Patentblatt 83/46**

(45) Bekanntmachung des Hinweises auf die Patenterteilung: **04.11.87 Patentblatt 87/45**

(84) Benannte Vertragsstaaten: **AT BE CH DE FR GB IT LI LU NL SE**

(56) Entgegenhaltungen:
**US-A-4 296 103**

**GMELINS "Handbuch der anorganischen Chemie", 8. Auflage, "Chlor" Ergänzungsband, Teil B, Lieferung 2, 1969, VERLAG CHEMIE, Weinheim/BergstraBe Seiten 400-401**
**A.F. HOLLEMANN "Lehrbuch der anorganischen Chemie", 57.-70 Auflage 1964, WALTER DE GRUYTER & CO., Berlin Seiten 123-127**
**Inorg. Nucl.Chem. Letters, Bd. 2, (1966), 395-398**

**Die Akte enthält technische Angaben, die nach dem Eingang der Anmeldung eingereicht wurden und die nicht in dieser Patentschrift enthalten sind.**

(73) Patentinhaber: **Kühne, Friedrich- Wilhelm Dr., Paulinenstrasse 56, D-7107 Neckarsulm (DE)**

(72) Erfinder: **Kühne, Friedrich- Wilhelm Dr., Paulinenstrasse 56, D-7107 Neckarsulm (DE)**

(74) Vertreter: **Beil, Walter, Dr., BEIL, WOLFF & BEIL Rechtsanwälte Adelonstrasse 58, D-6230 Frankfurt am Main 80 (DE)**

EP 0 093 875 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft den in den Patentansprüchen beschriebenen stabilisierten aktivierten Sauerstoff, der in einer Matrix aus Chloritionen eingeschlossen ist, und Arzneimittel, die diesen stabilisierten aktivierten Sauerstoff enthalten.

Sauerstoff ist bekanntlich die Voraussetzung für die Bildung von chemischer Energie in der lebenden Zelle, z. B. in Form der Bildung von Adenosintriphosphat. Sauerstoffmangel führt zu verschiedenen Mangelkrankheiten und Beschwerden des höheren Lebensalters. Darüberhinaus führt Sauerstoffmangel in den betroffenen Gewebebezirken auch zu einem starken Abfall des pH-Wertes.

Alle möglichen Schritte zur Stimulierung des Sauerstoff-Stoffwechsels der lebenden Zellen im Organismus zielen auf den langen Weg des Sauerstoffs zwischen der Sauerstoff-Diffusion aus den Lungenalveolen in das Lungenkapillarblut und seinem Metabolismus an den Cristea-Membranen der Mitochondrien in den Zellen.

Bekanntlich entstehen bei der Phagozytose oder bei Oberflächenkontakten durch Stimulierung des oxidativen Stoffwechsels, insbesondere des Hexosemonophosphatshunts der neutrophilen Granulozyten und der Makrophagen verschiedene hochaktive Sauerstoffverbindungen, die für die Keimabtötung von Bedeutung sind (vgl. Cottier, Pathogenese; ein Handbuch für die ärztliche Fortbildung, Bd. 2, S. 1289 bis 1292, Springer-Verlag 1980).

Aus der US-A-4 296 103 ist eine zur therapeutischen Anwendung geeignete stabilisierte wäßrige borhaltige Lösung von Chloroxiden bekannt, die durch Zusatz von 80 bis 120 Teilen Natriumchlorit, 90 bis 130 Teilen einer 13 %igen wäßrigen Natriumhypochloritlösung, 5 bis 10 $cm^3$ 37,7 %iger Salzsäure, 2 bis 4,5 $cm^3$ 95,15 %iger Schwefelsäure, 4 bis 15 Teilen Natrium- oder Kaliumperborat und 8 bis 15 Teilen Natriumperoxid zu 1000 Teilen Wasser in der angegebenen Reihenfäge erhalten wird. In diesem bekannten Verfahren wird keine Chlorylschwefelsäure zugesetzt, sondern es wird mit verhältnismäßig hohen Konzentrationen von Salzsäure und Schwefelsäure gearbeitet, wobei leicht ein explosives Gemisch von Chloroxiden gebildet wird. Das Herstellungsverfahren der US-A-4 296 103 führt nicht zur Bildung des Charge-Transfer-Komplexes $(Cl_2O_4)^-$ und somit auch nicht zur Bildung von stabilisiertem aktiviertem Sauerstoff, der in einer Matrix aus Chloritionen eingeschlossen ist.

Der Erfindung liegt die Aufgabe zugrunde, einen aktivierten Sauerstoff in stabilisierter Form als Produkt bereitzustellen.

Diese Aufgabe wird erfindungsgemäß durch einen stabilisierten aktivierten Sauerstoff, der in einer Matrix aus Chloritionen eingeschlossen ist, mit der allgemeinen Formel

$$ClO_2^- \times n\ O_2 \text{ (aktiviert)}$$

worin n den Wert 0,1 bis 0,25 bedeutet, einer Bande im Raman-Spektrum für aktivierten Sauerstoff bei 1 562 $cm^{-1}$ und einem O-O-Abstand von 123 pm gelöst.

Überraschenderweise kann mit Hilfe des erfindungsgemässen stabilisierten aktivierten Sauerstoffs zur Stimulierung des Sauerstoff-Stoffwechsels im Organismus der Weg über die Lungenalveolen und das Lungenkapillarblut umgangen werden, da der erfindungsgemäße Sauerstoff sich direkt am Sauerstoff-Transport beteiligt und das Hämoglobin in seiner Funktionsweise hervorragend und in bisher unerreichtem Maße beim Transport von Sauerstoff in die Zellen unterstützt. Er besitzt auch eine hohe physiologische Verträglichkeit. Darüberhinaus ist der erfindungsgemäße stabilisierte aktivierte Sauerstoff in der Lage, einen abgefallenen pH-Wert wieder zu normalisieren. Dadurch werden alle jene Prozesse beeinflußt, die mit der Zellmembran zusammenhängen, wie die Energieproduktion der Zelle, die immunologischen Prozesse oder Enzymreaktionen. Die Versteifung von Erythrozytenmembranen wird aufgehoben und die Erythrozytenflexibilität wieder hergestellt.

Der erfindungsgemäße stabilisierte aktivierte Sauerstoff, der in einer Matrix aus Chloritionen eingeschlossen ist, kann hergestellt werden, indem man ein Chlorit, wie Natriumchlorit, in wäßriger Lösung mit einem Hypochlorit, wie Natriumhypochlorit umsetzt. Die Umsetzung erfolgt in einem Molverhältnis von Chlorit zu Hypochlorit wie 1 : 0,2 bis 1 : 0,3, vorzugsweise 1 : 0,25. In diesem Medium erfolgt innerhalb einer Stunde keine Veränderung der Edukte. Diese alkalische Lösung von Natriumchlorit und Natriumhypochlorit wird mit "Chlorylschwefelsäure" auf einen pH-Wert von 6,4 bis 6,8 titriert. Dabei bilden sich Chlordioxid und ein Charge-Transfer-Komplex $(Cl_2O_4)^-$. Aus diesem Komplex wird sodann unter Zusatz von Peroxidcarbonaten oder Peroxidboraten, z. B. Natriumperborat oder Natriumpercarbonat, und anschließend Natriumperoxid Chlordioxid ausgetrieben. Dabei entsteht eine Matrix aus Chloritionen, die aktivierten Sauerstoff eingeschlossen enthält. Das gegebenenfalls als Nebenprodukt entstehende Borat wird später durch Auskristallisieren entfernt.

Die vorstehende Reaktionsfolge kann wie folgt erläutert werden:

$$2\ ClO_2^- + OCl^- + 2H^+ \rightarrow 2\ ClO_2 + Cl^- + H_2O \qquad (1)$$

$$ClO_2 + ClO_2^- \rightarrow (Cl_2O_4)^- \qquad (2)$$

Die Hälfte der eingesetzten Chloritmenge wird gemäß Gleichung (1) in einem Redoxprozeß zu $ClO_2$ oxidiert. Das entstehende Chlordioxid bildet mit der zweiten Hälfte nichtoxidiertem Chlorit einen intensiv braunen Charge-Transfer-Komplex entsprechend Gleichung (2).

Das Maximum an Ausbeute wird nach 60 bis 90 Minuten erreicht. Anschließend versetzt man die Lösung - je nach künftiger Verwendung - mit geringen Mengen $Na_2CO_3$ x $H_2O_2$ oder $NaBO_2$ x $H_2O_2$ x $3H_2O$, wobei sich die Farbe nach gelb aufhellt. Durch das Peroxid wird ein Teil des gelösten Chlordioxids unter stürmischer Entwicklung von Sauerstoff wieder zu Chlorit reduziert. Nach weiteren 15 Minuten gibt man eine geringe Menge $Na_2O_2$ zur Lösung, die sich nun vollständig entfärbt, da restliches $ClO_2$ zu $ClO_2^-$ reduziert wird. Nunmehr erfolgt in sehr langsamer Reaktion, die mindestens 4 Wochen erfordert und üblicherweise mehr als 6 Wochen in Anspruch nimmt, die Bildung einer aktivierten Sauerstoff enthaltenden Matrix aus Chloritionen unter gleichzeitiger Bildung von Hydroxylionen. Diese Reaktion kann nicht beschleunigt werden. Der pH-Wert erhöht sich bis auf 13,8. Verschiedene Versuche haben gezeigt, daß sich das Volumen über der Lösung auf den Sauerstoffgehalt auswirkt. Am günstigsten erwies sich ein Verhältnis von 2/3 Lösung zu 1/3 Gasvolumen.

Stöchiometrisch sollte man, von der gemäß Gleichung (1) gebildeten Menge Chlordioxid aus gesehen, ein Verhältnis von Chlorit zu Sauerstoff wie 4 : 1 erwarten. Hinzu kommt möglicherweise Sauerstoff, welcher sich durch Disproportionierung von Wasserstoffperoxid bildet. Das Verhältnis sollte sich also noch mehr zugunsten von Sauerstoff verschieben. Quantitative Sauerstoffbestimmungen haben aber gezeigt, daß so hohe Sauerstoff-Konzentrationen nicht möglich sind und überschüssiger Sauerstoff entweicht, weil einerseits die Löslichkeit von Sauerstoff in einer $ClO_2^-$-Matrix begrenzt ist, und andererseits sich ein beliebig hoher Sauerstoffpartialdruck nicht aufrechterhalten läßt und auch gar nicht erwünscht ist, da sonst ein erwünschter Peroxidabbau blockiert wird.

Die Anwesenheit von $ClO_2^+$-Ionen, welche sich mit $ClO_3^-$ in einem Gleichgewicht befinden,

$$OClO_2^- + 2\,HOSO_3H \rightleftarrows ClO_2^+ + 2\,SO_4H^- + H_2O \qquad (3)$$

lösen einen Animpfeffekt aus, der die Reaktion in Richtung von Chlordioxid lenkt:

$$ClO_2^+ + ClO_2^- \rightarrow 2\,ClO_2 \qquad (4)$$

Der möglichen Bildung von Chlorat wird dadurch entgegengesteuert.

Optimierungsversuche haben ein stöchiometrisches Verhältnis von $NaClO_2 : NaOCl = 1 : 0{,}21$ ergeben. Hier ist zwar die maximal zu erwartende $ClO_2$-Ausbeute noch nicht ganz erreicht, doch ist der $ClO_3^-$-Gehalt noch sehr gering. Zunehmende $OCl^-$-Konzentrationen bewirken zwar mehr $ClO_2$, doch geht dies Kosten vermehrter Chlorat-Bildung. Die Entstehung des hier zu erwartenden Charge-Transfer-Komplexes $[Cl_2O_4]^-$ wird im Raman-Spektrum vor allem durch die beiden Banden bei 947 und 802 $cm^{-1}$ angezeigt. Deren Intensitäten sollen etwa 1:1 (Optimum), Abweichungen hiervon nicht mehr als 25 %, betragen. Die Anwesenheit von nicht erwünschtem Chlorat macht sich durch eine Bande bei 937 $cm^{-1}$ bemerkbar (daneben auch Banden bei 487 und 618 $cm^{-1}$).

Die Normalpotentiale betragen für das saure Medium 1,5 V und für das alkalische Medium 0,85 V. Eine zu hohe Wasserstoffionen- oder Hypochlorit-Konzentration würde infolge der verstärkt oxidierenden Wirkung eine Chlorat-Bildung (Oxidation von $ClO_2^-$ zu $ClO_3^-$) begünstigen. Der Redoxprozess soll aber in Richtung Chlordioxid gelenkt werden: Die Normalpotentiale $e_0$ betragen für gasförmiges $ClO_2$ 1,15 V und für in Wasser gelöstes $ClO_2$ (vorliegend) in Form von $Cl_2O_4^-$ 0,95 V. Bei einem pH-Wert von 6,4 bis 6,8 liegt der $e_0$-Wert, also das Normalpotential für das System $OCl^-/Cl^-$, zwischen 0,85 und 1,5 V, also gerade ausreichend, um $ClO_2^-$ zu $ClO_2$ zu oxidieren und eine Chloratbildung zu unterdrücken.

Für das erhaltene Produkt aus aktiviertem Sauerstoff, der in einer Matrix aus Chloritionen eingeschlossen ist, läßt sich die allgemeine Formel

$$ClO_2^- \times n\,O_2 \text{ (aktiviert)}$$

worin n den Wert 0,1 bis 0,25 bedeutet, aufstellen.

Die experimentell ermittelten Sauerstoffgehalte bewegen sich um 75 ppm bei einer drei Jahre alten Lösung. Bei einer frisch bereiteten Lösung liegen die Werte anfangs bei über 200 ppm, um nach 4 Wochen auf den Endwert von 75 ppm zu fallen. Dieser Wert ist über Jahre stabil. Die Ergebnisse zeigen überraschend hohe Werte, wenn berücksichtigt wird, daß normalerweise bei einer Lösung mit zunehmendem Salzgehalt die Löslichkeit von Sauerstoff rapide absinkt.

Der erfindungsgemäß stabilisierte aktivierte Sauerstoff, der in einer Matrix aus Chloritionen eingeschlossen ist, kann auf verschiedenen Gebieten Anwendung finden, beispielsweise in der Medizin und Veterinärmedizin, in der Kosmetik, zum Sterilisieren von Lebensmitteln und Trinkwasser sowie als Futtermittelzusatz. Allgemeine medizinische Anwendungsbereiche liegen auf den Gebieten der Desinfektion und der Chemoprophylaxe. Speziell kann der erfindungsgemäße stabilisierte aktivierte Sauerstoff z. B. zur Behandlung von Hautschäden, wie Herpes simplex, Herpes zoster, Akne oder Verbrennungen, oder von Wundheilungsstörungen, oder auch zur Makrophagen- und Phagozytenstimulierung angewendet werden. Insbesondere kann eine Arteriopathie signifikant beeinflußt werden. Ferner kann die Alopecia geheilt werden. Bei Melanomen werden bis zu 50 %ige Remissionen erzielt.

Andere Anwendungsgebiete des erfindungsgemäßen stabilisierten aktivierten Sauerstoffs liegen auf technischem Gebiet. So kann der stabilisierte aktivierte Sauerstoff Mineralölprodukten zugesetzt werden, um bei der Verbrennung dieser Produkte eine bessere Energieausbeute zu erreichen.

**Beispiel 1**

In einem geschlossenen Gefäß wurde eine Lösung von 106,4 g (1 Mol) Natriumchlorit in 1 l destilliertem Wasser mit 92,8 ml einer Natriumhypochloritlösung mit einem Gehalt an aktivem Chlor von 13 %, entsprechend 0,21 Mol Natriumhypochlorit versetzt. Diese Lösung, die einen pH-Wert von 10,4 aufwies, wurde unter vorsichtigem Rühren tropfenweisie mit 5,3 ml einer Chlorylschwefelsäure (hergestellt durch Zusatz von 157,35 mg $KClO_3$ zu 122,5 ml Schwefelsäure einer Dichte von 1,6) entsprechend 6,81 mg (55,6 x $10^{-6}$ Mol) $KClO_3$ als Katalysator auf einen pH-Wert von 6,4 bis 6,8 titriert. Nach einer Reaktionszeit von 90 Minuten wurde die Lösung vorsichtig mit 6 g (0,039 Mol) Natriumperborat ($NaBO_2$ x $H_2O_2$ x 3 $H_3O$) versetzt, und anschließend wurde unter kräftigem Rühren Chlordioxid ausgetrieben. Nach einer kräftigen Reaktion für die Dauer von 15 Minuten wurde das erhaltene Reaktionsgemisch mit 12 g (0,154 Mol) Natriumperoxid ($Na_2O_2$) versetzt. Die dabei auftretende exotherme Reaktion wurde durch stetes Rühren unterbunden. Nach etwa 6 Wochen wurde das Produkt in Form einer wäßrigen Lösung eines Komplexes aus aktiviertem $O_2$ und $ClO_2^-$ von dem ausgefallenen Natriumborat abfiltriert.

Die erhaltene Lösung hatte einen pH-Wert zwischen 13,5 und 13,8, aber keinen Laugencharakter. Sie stellte eine wasserklare, mit Alkoholen mischbare Flüssigkeit vom Schmelzpunkt -3°C und einer Leitfähigkeit von 3,45 mS · $cm^{-1}$ dar. Im Raman-Spektrum wies sie im wesentlichen Banden bei 403, 802 (Chlorit) und 1562 $cm^{-1}$ (aktivierter Sauerstoff) auf. Zur Bestimmung der zuletzt genannten Bande wurde, da die 02-Bande im Raman-Spektrum teilweise durch die $\delta(OH_2)$-Bande verdeckt wurde, ein Ansatz gemäß vorstehender Arbeitsweise unter Verwendung von $D_2O$ durchgeführt. Im so erhaltenen Konzentrat konnte die $O_2$-Bande eindeutig bei 1562 $cm^{-1}$ festgelegt werden, da $\delta(OD_2)$ wegen des Isotopeneffektes bei niedrigeren Wellenzahlen auftritt. Der O-O-Abstand des sich in der Chlorit-Matrix befindlichen, aktivierten Sauerstoffs wurde zu 123 pm berechnet. Damit war diese Bindung gegenüber dem nichtaktivierten Sauerstoff (120 pm) merklich verlängert.

Mittels Hochdruckflüssigkeitschromatographie ergab sich ein charakteristischer RT-Wert für den Absorptionspeak bei 196 mm von 2,14, der sich deutlich von Peroxid, Chlorit, Hypochlorit und Chlorat unterscheidet.

**Beispiel 2**

Die Arbeitsweise von Beispiel 1 wurde wiederholt, wobei jedoch anstelle der 0,039 Mol Natriumperborat 0,049 Mol Natriumpercarbonat eingesetzt wurden. Das erhaltene Produkt entsprach dem produkt von Beispiel 1.

**Beispiel 3**

**Arzneimittel**
Aus dem produkt von Beispiel 1 wurde durch Verdünnen mit destilliertem Wasser im Verhältnis 1:50 eine Arzneimittellösung hergestellt, die einen pH-Wert von 11,45 bis 11,6 aufwies und physiologisch verträglich war.

**Patentansprüche** für die Vertragsstaaten BE, CH, DE, FR, GB, IT, LU, NL, SE

1. Stabilisierter aktivierter Sauerstoff, der in einer Matrix aus Chloritionen eingeschlossen ist, mit der allgemeinen Formel

$ClO_2^-$ x n $O_2$ (aktiviert)

worin n den Wert 0,1 bis 0,25 bedeutet, einer Bande im Raman-Spektrum für aktivierten Sauerstoff bei 1 562 $cm^{-1}$ und einem O-O-Abstand von 123 pm.

2. Stabilisierter Sauerstoff nach Anspruch 1, dadurch gekennzeichnet, daß er erhältlich ist durch Umsetzung eines Chlorits in wäßriger Lösung mit einem Hypochlorit im Molverhältnis von Chlorit zu Hypochlorit von 1:0,2 bis 1:0,3, Titrieren der erhaltenen alkalischen Lösung mit Chlorylschwefelsäure auf einen pH-Wert von 6,4 bis 6,8 unter Bildung von Chlordioxid und des Charge-Transfer-Komplexes $(Cl_2O_4)^-$, Austreiben von Chlordioxid aus diesem Komplex unter Zusatz von Feroxidcarbonaten oder Peroxidboraten, Zusatz von Natriumperoxid, Stehenlassen des Reaktionsgemisches für die Dauer von mindestens 4 Wochen und Abtrennen der den Komplex aus aktiviertem Sauerstoff und Chloritionen enthaltenden wäßrigen Lösung von dem ausgefallenen Carbonat oder Borat.

3. Stabilisierter Sauerstoff nach Anspruch 1 oder 2, dadurch gekennzeichnet, daß er in Form einer konzentrierten Lösung mit einem pH-Wert von 13,5 bis 13,8 vorliegt.

4. Stabilisierter Sauerstoff nach Anspruch 1 oder 2, dadurch gekennzeichnet, daß er in Form einer Lösung mit

einem pH-Wert von 11,45 bis 11,6 vorliegt.

5. Stabilisierter Sauerstoff nach einem der Ansprüche 3 oder 4, dadurch gekennzeichnet, daß die Lösung Natrium- und Kaliumionen im Verhältnis 30:1 enthält.

6. Arzneimittel, gekennzeichnet durch einen Gehalt an stabilisiertem aktiviertem Sauerstoff nach einem der Ansprüche 1 bis 5.

**Patentansprüche** für den Vertragsstaat AT

1. Verfahren zur Herstellung von stabilisiertem, aktivierten Sauerstoff, der in einer Matrix aus Chloritionen eingeschlossen ist, mit der allgemeinen Formel

$ClO_2^- \times n\ O_2$ (aktiviert)

worin n den Wert 0,1 bis 0,25 bedeutet, einer Bande im Raman-Spektrum für aktivierten Sauerstoff bei 1 562 $cm^{-1}$ und einem O-O-Abstand von 123 pm, dadurch gekennzeichnet, daß man ein Chlorit in wäßriger Lösung mit einem Hypochlorit im Molverhältnis von Chlorit zu Hypochlorit von 1:0,2 bis 1:0,3 umsetzt, die erhaltene alkalische Lösung mit Chlorylschwefelsäure auf einen pH-Wert von 6,4 bis 6,8 unter Bildung von Chlordioxid und des Charge-Transfer-Komplexes $(Cl_2O_4)^-$ titriert, aus diesem Komplex unter Zusatz von Peroxidcarbonaten oder Peroxidboraten Chlordioxid austreibt, Natriumperoxid zusetzt, das Reaktionsgemisch für die Dauer von mindestens 4 Wochen stehenläßt und die den Komplex aus aktiviertem Sauerstoff und Chloritionen enthaltende wäßrige Lösung von dem ausgefallenen Carbonat oder Borat abtrennt.

2. Verfahren nach Anspruch 1, dadurch gekennzeichnet, daß man den Komplex aus aktiviertem Sauerstoff und Chloritionen in Form einer konzentrierten wäßrigen Lösung mit einem pH-Wert von 13,5 bis 13,8 erhält.

3. Verfahren nach Anspruch 2, dadurch gekennzeichnet, daß man die konzentrierte wäßrige Lösung mit destilliertem Wasser auf einen pH-Wert von 11,45 bis 11,6 verdünnt.

4. Verfahren zur Herstellung eines Arzneimittels mit einem Gehalt an stabilisiertem, aktivierten Sauerstoff, der in einer Matrix aus Chloritionen eingeschlossen ist, mit der allgemeinen Formel

$ClO_2^- \times n\ O_2$ (aktiviert)

worin n den Wert 0,1 bis 0,25 bedeutet, einer Bande im Raman-Spektrum für aktivierten Sauerstoff bei 1 562 $cm^{-1}$ und einem O-O-Abstand von 123 pm, dadurch gekennzeichnet, daß man die nach dem Verfahren von Anspruch 1 oder 2 erhaltene wäßrige Lösung mit destilliertem Wasser auf einen pH-Wert von 11,45 bis 11,6 verdünnt.

**Claims** for the contracting States BE, CH, DE, FR, GB, IT, LU, NL, SE.

1. Stabilized activated oxygen incorporated in a matrix of chlorite ions and having the following general formula

$ClO_2^- \times n\ O_2$ (activated)

in which n has the value 0.1 to 0.25,
a band in the Raman spectrum for activated oxygen at 1562 $cm^{-1}$ and an O-O-interval of 123 pm.

2. Stabilized oxygen as claimed in Claim 1, characterized in that it is obtainable by reacting a chlorite in aqueous solution with a hypochlorite in a molar ratio of chlorite to hypochlorite of from 1:0.2 to 1:0.3, titrating the alkaline solution obtained with chloryl sulfuric acid to a pH value of from 6.4 to 6.8 with formation of chlorine dioxide and the charge-transfer complex $(Cl_2O_4)^-$, removing chlorine dioxide from this complex with addition of peroxycarbonates or peroxyborates, adding sodium peroxide, allowing the reaction mixture to stand for at least 4 weeks and separating the aqueous solution containing the complex of activated oxygen and chlorite ions from the carbonate or borate precipitated.

3. Stabilized oxygen as claimed in Claim 1 or 2, characterized in that it is in the form of a concentrated solution having a pH value of from 13.5 to 13.8.

4. Stabilized oxygen as claimed in Claim 1 or 2, characterized in that it is in the form of a solution having a pH value of from 11.45 to 11.6.

5. Stabilized oxygen as claimed in Claim 3 or 4, characterized in that the solution contains sodium and potassium ions in a ratio of 30:1.

6. A medicament characterized by a content of the stabilized activated oxygen claimed in any of Claims 1 to 5.

**Claims** for the contracting State AT

1. A process for the production of stabilized activated oxygen incorporated in a matrix of chlorite ions and having the following general formula

$ClO_2^- \times n\ O_2$ (activated)

in which n has the value 0.1 to 0.25,
a band in the Raman spectrum for activated oxygen at 1562 $cm^{-1}$ and an O-O-interval of 123 pm,
characterized in that a chlorite is reacted in aqueous solution with à hypochlorite in a molar ratio of chlorite to hypochlorite of from 1:0.2 to 1:0.3, the alkaline solution obtained is titrated with chloryl sulfuric acid to a pH value of from 6.4 to 6.8 with formation of chlorine dioxide and the charge-transfer complex $(Cl_2O_4)^-$, chlorine dioxide is removed from this complex with addition of peroxycarbonates or peroxyborates, sodium peroxide is added, the reaction mixture is left standing for at least 4 weeks and the aqueous solution containing the complex of activated oxygen and chlorite ions is separated from the carbonate or borate precipitated.

2. A process as claimed in Claim 1, characterized in that the complex of activated oxygen and chlorite ions is obtained in the form of a concentrated aqueous solution having a pH value of from 13.5 to 13.8.

3. A process as claimed in Claim 2, characterized in that the concentrated aqueous solution is diluted with distilled water to a pH value of from 11.45 to 11.6.

4. A process for the preparation of a medicament containing stabilized activated oxygen incorporated in a matrix of chlorite ions and having the following general formula

$ClO_2^- \times n\ O_2$ (activated)

in which n has the value 0.1 to 0.25,
a band in the Raman spectrum for activated oxygen at 1562 $cm^{-1}$ and an O-O-interval of 123 pm, characterized in that the aqueous solution obtained by the process claimed in Claim 1 or 2 is diluted with distilled water to a pH value of from 11.45 to 11.6.

**Revendications** pour les Etats contractants: BE, CH, DE, FR, GB, IT, LU, NL, SE.

1. Oxygène activé stabilisé, inclus dans une matrice d'ions chlorite, ayant la formule générale

$ClO_2^- \times n\ O_2$ (activé)

dans laquelle n vaut de 0,1 à 0,25, avec une bande au spectre Raman de 1562 $cm^{-1}$ pour l'oxygène activé, et une distance O-O de 123 pm.

2. Oxygène stabilisé selon la revendication 1, caractérisé en ce qu'on l'obtient par la réaction d'un chlorite en solution aqueuse sur un hypochlorite, selon un rapport en moles entre le chlorite et l'hypochlorite de 1:0,2 à 1:0,3, titrage de la solution alcaline obtenue avec de l'acide chlorylsulfurique à un pH de 6,4 à 6,8 avec formation d'un dioxyde de chlore et du complexe à transfert de charge $(Cl_2O_4)^-$, expulsion du dioxyde de chlore à partir de ce complexe, par addition de peroxycarbonates ou de peroxyborates, addition de peroxyde de sodium, abandon au repos du mélange réactionnel pendant au moins 4 semaines, et séparation, d'avec le carbonate ou le borate précipité, de la solution aqueuse contenant le complexe constitué d'oxygène activé et d'ions chlorite.

3. Oxygène stabilisé selon les revendications 1 ou 2, caractérisé en ce qu'il se présente sous la forme d'une solution concentrée ayant un pH de 13,5 à 13,8.

4. Oxygène stabilisé selon la revendication 1 ou 2, caractérisé en ce qu'il se présente sous la forme d'une solution ayant un pH de 11,45 à 11,6.

5. Oxygène stabilisé selon l'une des revendications 3 ou 4, caractérisé en ce que la solution contient des ions sodium et potassium selon le rapport 30:1.

6. Médicament, caractérisé en ce qu'il contient un oxygène activé stabilisé selon l'une des revendications 1 à 5.

**Revendications** pour l'Etat contractant: AT.

1. Procédé pour préparer de l'oxygène activé stabilisé, inclus dans une matrice d'ions chlorite, ayant la formule générale

$ClO_2^- \times n\ O_2$ (activé)

ou n vaut de 0,1 à 0,25, avec une bande à 1562 $cm^{-1}$ pour l'oxygène activé au spectre Raman et une distance O-O de 123 pm, caractérisé en ce qu'on fait réagir un chlorite en solution aqueuse sur un hypochlorite, avec un rapport en moles entre le chlorite et l'hypochlorite de 1:0,2 à 1:0,3, qu'on titre la solution alcaline obtenue avec de l'acide chlorylsulfurique à un pH de 6,4 à 6,8 avec formation de dioxyde de chlore et du complexe à transfert de charge $(Cl_2O_4)^-$, qu'on expulse du dioxyde de chlore de ce complexe, sous addition de peroxycarbonates ou de peroxyborates, qu'on ajoute du peroxyde de sodium, qu'on laisse reposer le mélange réactionnel pendant au moins 4 semaines, et qu'on sépare du carbonate ou du borate précipité la solution aqueuse contenant le complexe constitué d'oxygène activé et d'ions chlorite.

2. Procédé selon la revendication 1, caractérisé en ce qu'on obtient le complexe constitué d'oxygène activé et d'ions chlorite sous la forme d'une solution aqueuse concentrée ayant un pH de 13,5 à 13,8.

3. Procédé selon la revendication 2, caractérisé en ce qu'on dilue la solution aqueuse concentrée, avec de l'eau distillée, à un pH de 11,45 à 11,6.

4. Procédé de préparation d'un médicament contenant de l'oxygène activé stabilisé inclus dans une matrice d'ions chlorite, ayant la formule générale

$ClO_2^-$ x n $O_2$ (activé)

où n vaut de 0,1 à 0,25, avec une bande à 1562 $cm^{-1}$ au spectre Raman pour l'oxygène activé et une distance O-O de 123 pm, caractérisé en ce qu'on dilue à un pH de 11,45 à 11,6, à l'eau distillée, la solution aqueuse obtenue par le procédé de la revendication 1 ou 2.